# EUROPEAN PATENT APPLICATION

(11) **EP 4 163 392 A1**
(43) Date of publication of application: **12.04.2023**
(21) Application number: 21306403.3
(22) Date of filing: 06.10.2021
(51) Int. Cl.: C12Q 1/6883

(54) **METHODS AND KITS FOR DIAGNOSING MUSCLE ATROPHY**

(71) Applicant: Institut national de recherche pour l'agriculture, l'alimentation et l'environnement, 75007 Paris (FR); Université Clermont Auvergne, 63001 Clermont-Ferrand (FR); Centre Hospitalier Universitaire de Clermont Ferrand, 63003 Clermont-Ferrand (FR)
(72) Inventor: TAILLANDIER, Daniel, 63122 St Genes Champanelle (FR); HENG, Anne Elisabeth, 63000 Clermond-Ferrand (FR); ANIORT, Julien, 63000 Clermond-Ferrand (FR)
(74) Representative: Cabinet Becker et Associés

(57) **Abstract**

The present invention relates to compositions and methods for detecting muscle atrophy in a subject. The invention also provides novel biomarkers that allow *in vitro* or *ex vivo* detection of a loss of muscle mass in a subject. The invention also relates to the use of muscle atrophy as an indicator of the occurrence, or the risk of occurrence of a disease or a disorder in a subject. Furthermore, the invention relates to a kits and reagents for use in these methods.

## Description

### FIELD OF THE INVENTION

The present invention relates to compositions and methods for detecting muscle atrophy in a subject. The invention also provides novel biomarkers that allow *in vitro* or *ex vivo* detection of muscle mass loss in a subject. The invention also relates to the use of muscle atrophy as an indicator of the occurrence, or the risk of occurrence of a disease or a disorder in a subject, including addressing the potential deleterious effect of some therapies and the effectiveness of potential countermeasures. Furthermore, the invention relates to kits and reagents, such as oligonucleotides, for use in these methods.

### BACKGROUND OF THE INVENTION

Skeletal muscle accounts for nearly half of the mass of the human body. In addition to its role in locomotion, metabolism, homeostasis and storage of energy and nitrogen, skeletal muscle (which contains 50-75% of all proteins in the human body) is an essential supplier of amino acids to be used by vital organs in the event of a pathology.

Malnutrition, muscle disuse, aging, and illness or injury cause skeletal muscle fibers to become atrophic, ultimately leading to a loss of muscle mass. This process, called skeletal muscle atrophy, is associated with impaired quality of life, decreased tolerance and therapy response, and increased morbidity and mortality whatever the causal disease (Kalantar-Zadeh et al., J Cachexia Sarcopenia Muscle (2013) 4:89-94). When skeletal muscle atrophy has been established for a long time, it is very difficult to reverse. Thus, it appears essential to predict early in the disease that muscle atrophy is taking hold.

Skeletal muscle homeostasis is controlled by numerous signaling pathways. The decrease in muscle mass is due to an imbalance in protein homeostasis in favor of proteolysis. The comparison of the gene expression profiles of muscles from different catabolic conditions enabled the identification of a common panel of genes, named atrogenes, which are thought to be important for protein loss. Several atrogenes belong to the two major cellular degradation systems, the ubiquitin-proteasome and autophagy-lysosome. In rodent atrophying muscles, the two ubiquitin ligases muscle ring finger-1 (MuRF1/TRIM63) and muscle atrophy F-box (MAFbx/Atrogin-1) are considered the master genes of muscle atrophy (Bodine et al., Science (2001) 294(5547):1704-1708) while TRIM32 appears as a master regulator of muscle renewal through the initiation of autophagy (Overå et al., J Cell Sci. (2019) 132(23): jcs236596).

After the discovery of the atrogenes, the concept that transcription factors drive muscle atrophy is now well-established (Sartori et al., Nature Communications (2021) 12:330). FoxO1,3,4 are transcription factors downstream the IGF1/insulin-Akt pathway and their inhibition completely spares muscle loss and force drop in fasting, hind limb suspension, immobilization, diabetes, and glucocorticoids treatment. NF-κB and Stat3 transcription factors mediate the effect of TNF-α and IL-6 on muscle wasting, in particular by upregulating either MuRF1 or MAFbx. Unfolded protein response (UPR) and endoplasmic reticulum (ER)-stress-related pathways have been found to regulate muscle atrophy via ATF4 and XBP1 transcription factors.

Muscle atrophy can also be associated with muscle-specific disease. Although less intense than disuse-associated muscle atrophy, disease-associated muscle atrophy can result from diseases that either affect the nerves supplying individual muscles (i.e., neurogenic atrophy) or from diseases intrinsic to muscle tissue (i.e., muscle disease). In neurogenic atrophy, the nerve supply to the muscle can be interrupted or compromised by compression, injury, or disease within the nerve cells, resulting in a temporary or permanent nerve deficit. Diseases within nerve cells that can interrupt or compromise nerve supply to muscles include, for example, multiple sclerosis, amyotrophic lateral sclerosis (ALS, or Lou Gehrig's disease), Guillain-Barre syndrome, stroke, and viral infection of nerve cells (e.g., poliomyelitis). Muscle diseases can be intrinsic to muscle tissue (e.g., muscular dystrophy, polymyositis, or myotonia) or can occur as a response to systemic illness (e.g., hypo- or hyperthyroidism, adrenal gland depletion, diabetes mellitus, or autoimmune diseases). Sarcopenia is a debilitating disease that afflicts the elderly and is characterized by loss of muscle mass and function with advanced age.

Generalized muscle wasting (cachexia) can also occur as a secondary consequence of diseases such as advanced cancer, Acquired Immune Deficiency Syndrome (AIDS), chronic obstructive lung disease, congestive heart failure, cardiomyopathy, chronic liver disease, renal disease, emphysema, tuberculosis, osteomalacia, hormonal deficiency, anorexia nervosa, generalized malnutrition, and drug abuse (e.g., abuse of alcohol, opiates, or steroids). Skeletal muscle atrophy in cachexia cannot be reversed by increasing food intake in the absence of the treatment of the causal pathology (Konishi et al., J Cachexia Sarcopenia (2016) 7:107-109). Mechanical stress and neuro-hormonal mediators are also involved in the control of muscle mass during cachexia. Studies on the mechanisms of muscle wasting are mostly conducted in animal models, such as young growing rodents. Although some abnormalities described in animal models have been confirmed in humans, it still remains to be established whether the modifications observed are directly linked to the atrophying program or whether they are specific to the disease itself. Moreover, muscle wasting is generally detected within 1-2 weeks after catabolic stimuli in young rodents, whereas in human diseases it is generally detected within longer periods and in adults. In addition, the physiology of rodents is not strictly comparable with that of humans.

Recently, inventors conducted studies in atrophying skeletal muscle biopsies from lung cancer (LC) and chronic haemodialysis (HD) patients (Aniort et al., J Cachexia Sarcopenia Muscle (2019) 10(2): 323-337). Both the ubiquitin proteasome and autophagy systems are activated in LC and HD patients and some already known E3 ligases were confirmed as potent markers of human muscle atrophy. Wnt-β-catenin and ATF4 signaling pathways were also activated in patients. A panel of more than 230 proteins are differentially expressed in LC and HD patients when compared to the healthy human proteome. However, protein variations were not addressed at the transcriptomic level. Moreover, mass spectrometry analysis of protein is hardly useful for direct clinical use. In addition, muscle biopsies are painful, difficult to access to, and not appropriate for monitoring muscle atrophy over time.

Thus, there is a strong need for identifying biomarkers of muscle atrophy, from easily accessed samples, having good sensitivity and specificity independently of the disease. Such biomarkers may be highly valuable for diagnosing muscle atrophy in a subject, in particular for monitoring a loss of muscle mass and for helping in managing treatment in a subject suffering from a disease or disorder.

### SUMMARY OF THE INVENTION

The present invention is based on the identification of novel biomarkers detectable in fluids such as blood, which allow diagnosis of muscle atrophy, in particular skeletal muscle atrophy. By identifying a restricted panel of genes in biological samples from diseased subjects suffering from loss in muscle mass, the inventors have developed a simple and reliable method for diagnosing and monitoring muscle atrophy. Expression of these genes is highly correlated to the atrophying program whatever the disease. The invention thus allows to adjust and improve the subject treatment, by treating or reducing muscular atrophy in the management of the pathology. In particular, the detection and treatment of muscular atrophy in a diseased subject makes it possible to increase the effectiveness of a therapeutic treatment.

The present invention relates to the use of at least one gene or its gene product selected from ACTR6 (SEQ ID NO:1), GIMAP2 (SEQ ID NO:2), RCN2 (SEQ ID NO:3), RPL22L1 (SEQ ID NO:4), TRIAP1 (SEQ ID NO:5), NIFK (SEQ ID NO:6), APIP (SEQ ID NO:7), GEMIN6 (SEQ ID NO:8), RWDD1 (SEQ ID NO:9), ZNF613 (SEQ ID NO: 10), BPNT1 (SEQ ID NO:11), CCT2 (SEQ ID NO:12) and LYRM2 (SEQ ID NO:13) as a biomarker for *in vitro* or *ex vivo* detection of muscle atrophy, in particular skeletal muscle atrophy.

Another object of the invention relates to an *in vitro* or *ex vivo* method for diagnosing muscle atrophy, in particular skeletal muscle atrophy, in a subject, typically comprising determining the expression level of at least one biomarker selected from ACTR6, GIMAP2, RCN2, RPL22L1, TRIAP1, NIFK, APIP, GEMIN6, RWDD1, ZNF613, BPNT1, CCT2 and LYRM2 in a biological sample obtained from the subject; wherein a reduction in expression level of said at least one biomarker as compared to a reference value is indicative of muscle atrophy, in particular skeletal muscle atrophy, in the subject.

The present invention also relates to an *in vitro* or *ex vivo* method for assessing the efficacy of a treatment to reverse muscle atrophy, in particular skeletal muscle atrophy, in a subject. This method typically comprises the steps of:
(i) determining the expression level of at least one biomarker selected from ACTR6, GIMAP2, RCN2, RPL22L1, TRIAP1, NIFK, APIP, GEMIN6, RWDD1, ZNF613, BPNT1, CCT2 and LYRM2 in a first biological sample from the subject obtained prior to said treatment or at an early stage of said treatment; and
(ii) determining the expression level of the same at least one biomarker in a second biological sample from the subject obtained during, at a later stage, or after said treatment; wherein an expression level of the at least one biomarker as measured in step (ii) above the expression level of the same at least one biomarker as measured in step (i) is indicative that the treatment is effective.

The present invention further relates to an *in vitro* or *ex vivo* method for assessing the occurrence or the risk of occurrence of a disease in a subject, comprising assessing the presence of muscle atrophy, in particular skeletal muscle atrophy, by a method herein described; wherein the presence of muscle atrophy, in particular skeletal muscle atrophy, is indicative of the occurrence or the risk of occurrence of a disease in the subject.

In another aspect, the present invention relates to an *in vitro* or *ex vivo* method for helping in selecting, adapting or changing treatment in a subject suffering from a disease. This method typically comprises the steps of:
(i) assessing the presence of muscle atrophy, in particular skeletal muscle atrophy, in the subject by a method herein described; and
(iii) taking into account the presence of muscle atrophy, in particular skeletal muscle atrophy, for selecting, adapting or changing treatment in the subject suffering from a disease.

In a preferred aspect, the *in vitro* or *ex vivo* method for helping in selecting, adapting or changing treatment in a subject suffering from a disease further comprises a step (iii) of selecting a therapy for muscle atrophy, in particular skeletal muscle atrophy, wherein said therapy typically comprises drugs (pharmacological agents such as glucocorticoids, anti-inflammatory drugs), nutritional agents (such as dietary supplements, ursolic acid, tomatidine), exercise, or a combination thereof.

The invention may be used on a mammal, preferably a human subject, even more preferably an adult human subject.

In various embodiments of the methods of the present invention, the measure of the expression level of the biomarkers may be determined at the nucleic acid level, in particular RNA or cDNA, using well known techniques, such as for example, real time reverse transcriptase polymerase chain reaction (RT-PCR), quantitative real time reverse transcriptase polymerase chain reaction (qRT-PCR), digital PCR, RNAseq, microarrays, gene chips, nCounter Gene Expression Assay, Serial Analysis of Gene Expression (SAGE), Rapid Analysis of Gene Expression (RAGE), nuclease protection assays, Northern blotting, or any other equivalent gene expression detection techniques.

In other embodiments of the methods of the present invention, the measure of the expression level of the biomarkers may be determined at the protein level, using for example immunoassay, immunosorbent assay (ELISA), radioimmunoassay (RIA), mass spectrometry, western blotting, flow cytometry, or any other equivalent protein detection techniques.

The invention may be used on a fluid sample, preferably selected from peripheral blood, plasma, and serum.

In another aspect, the present invention relates to oligonucleotides having a nucleic acid sequence selected from the group consisting of SEQ ID NOs: 14-39. The oligonucleotides may be used as primer, in particular primer pairs, wherein each pair comprises a forward primer and a reverse primer specific for amplifying one of the target biomarkers.

In order to implement the methods of the invention, the above mentioned oligonucleotides primer pairs may be part of a device, such as for example a column, an array or a microarray; or a kit.

In a further aspect, the present invention relates to a kit comprising at least two oligonucleotides primers herein described for amplifying at least one biomarker selected from ACTR6, GIMAP2, RCN2, RPL22L1, TRIAP1, NIFK, APIP, GEMIN6, RWDD1, ZNF613, BPNT1, CCT2 and LYRM2, and optionally reagents for performing amplification reaction and/or instructions for use.

The present invention also relates to the use of a device or a kit on a biological sample obtained from a subject for diagnosing muscle atrophy, in particular skeletal muscle atrophy; for assessing the efficacy of a treatment to reverse muscle atrophy, in particular skeletal muscle atrophy; for assessing the occurrence or the risk of occurrence of a disease in a subject and/or for helping in selecting, adapting or changing treatment in a subject suffering from a disease.

The invention also relates to a method for diagnosing and treating muscle atrophy, in particular skeletal muscle atrophy, in a subject, comprising:
(i) determining the level of expression of at least one biomarker selected from ACTR6, GIMAP2, RCN2, RPL22L1, TRIAP1, NIFK, APIP, GEMIN6, RWDD1, ZNF613, BPNT1, CCT2 and LYRM2 in a biological sample obtained from the subject; wherein a reduction in expression level the at least on one biomarker as compared to a reference value is indicative of muscle atrophy, in particular skeletal muscle atrophy in the subject; and
(ii) treating the subject identified in step (i) as having muscle atrophy, in particular skeletal muscle atrophy, with drugs (pharmacological agents such as glucocorticoids, anti-inflammatory drugs), nutritional agents (such as dietary supplements, ursolic acid, tomatidine), exercise, or a combination thereof.

### BRIEF DESCRIPTION OF THE FIGURES

**Figure 1A****:** sPLS-DA was performed using the qRT-PCR data of both the PROMETHE and PHD patients, including 4 outliers. Healthy volunteers and patients with atrophying muscle (hemodialysis or lung cancer) are positioned in the space defined by the 2 first components of the sPLS-DA model. Patients with catabolic state appear as a distinct population from healthy volunteers.
**Figure 1B****:** Using the same data used in Figure 1A, sPLS-DA was performed using the qRT-PCR data of both the PROMETHE and PHD patients but without 4 outliers.
**Figure 2A****:** A ROC analysis was performed using the qRT-PCR data of both the PROMETHE and PHD patients, using a 1-component model. ACTR6 and GIMAP2 best predicted the 1-component model. The area under the ROC curve (AUC) = 0.9736.
**Figure 2B**: A ROC analysis was performed using the qRT-PCR data of both the PROMETHE and PHD patients, using a 2-components model. NIFK, RPL22L1, CCT2, GEMIN6 and RCN2 predicted the 2-components model. The area under the ROC curve (AUC) = 0.9934.

### DETAILED DESCRIPTION OF THE INVENTION

Skeletal muscle atrophy occurs in response to a variety of conditions including sepsis, glucocorticoid administration, fasting, muscle disuse, chronic kidney disease, diabetes, renal failure, cancer, HIV/AIDS, uremia, and numerous other chronic or systemic diseases.

Knowing the diversity of genes and proteins affecting loss of muscle mass, identifying and selecting relevant biomarkers allowing the diagnosis, in a subject, of muscle atrophy, in particular skeletal muscle atrophy, is a challenge. Identifying specific biomarkers directly linked to the atrophying program and that are unrelated to the disease or disorder itself is even more complex.

The invention is based on the identification of specific and highly reliable biomarkers of muscle atrophy, preferably skeletal muscle atrophy. These markers are ACTR6, GIMAP2, RCN2, RPL22L1, TRIAP1, NIFK, APIP, GEMIN6, RWDD1, ZNF613, BPNT1, CCT2, LYRM2 and/or their variants. These markers are detectable in fluid samples, such as blood samples, of patients, and are reliable and predictive of muscle atrophy regardless of the disease or disorder affecting said patient.

In a first aspect, the present invention relates to the use of at least one gene or its gene product selected from ACTR6 (SEQ ID NO:1), GIMAP2 (SEQ ID NO:2), RCN2 (SEQ ID NO:3), RPL22L1 (SEQ ID NO:4), TRIAP1 (SEQ ID NO:5), NIFK (SEQ ID NO:6), APIP (SEQ ID NO:7), GEMIN6 (SEQ ID NO:8), RWDD1 (SEQ ID NO:9), ZNF613 (SEQ ID NO:10), BPNT1 (SEQ ID NO:11), CCT2 (SEQ ID NO:12) and LYRM2 (SEQ ID NO:13) as a biomarker for *in vitro* or *ex vivo* detection of muscle atrophy, in particular skeletal muscle atrophy.

The present invention provides a biomarker determination method useful for diagnosing muscle atrophy, in particular skeletal muscle atrophy, by using a change in the expression level of at least one target gene as recited above, in a biological sample from a subject. The change in the expression level of at least one biomarker is generally obtained by comparing to a reference value.

### Definitions

The term "gene" designates a DNA sequence (whose component segments do not necessarily need to be physically contiguous) that specifies one or more sequence-related RNAs/proteins. As intended in the present invention, genes may be in the form of a sequence of only the protein-encoding portion of the sequences of the respective genes (cDNA), and may include a sequence other than the portion encoding the protein (DNA). In all humans, variations in genetic material lead to gene polymorphism. "Gene polymorphism" is an individual difference of the nucleotide sequence of DNA that constitutes a gene. In general, it is defined to appear at a collective frequency of 1% or greater. Single nucleotide polymorphisms (SNPs), which are defined as a change that can occur in one of every 1000 base pairs, are common in the human genome, can be located in different region of the gene such as for example promoter, coding or non-coding region, and also in intergenic region.

The expression "gene product" refers to what is transcribed or translated from the gene in question, such as an mRNA or a protein. The different isoforms or variants of mRNA and the resulting protein isoforms or variants are contemplated within the term gene product. Fragments of a gene product are also contemplated, as long as they are functionally active.

As used herein, the term "biomarker" refers to a biological molecule whose presence and/or concentration can be detected and correlated typically with a condition of interest. A biomarker can be a polypeptide or its coding nucleic acid (e.g., mRNA). In the context of the present invention, the biomarker can be used to classify a sample from a subject as a "muscle atrophy" sample, to assess the severity of the muscle atrophy, to assess the efficiency of a treatment in a diseased subject, and/or to assess the occurrence or the risk of occurrence of a disease in a subject suffering from muscular atrophy.

The term "atrophy" defines a decrease in the size of a tissue or organ due to cellular shrinkage and/or cell number decrease. The decrease in cell size is caused by loss of organelles, proteins and cytoplasm.

The expression "muscle atrophy" designates a loss of muscle mass, including the loss of muscle fibers and/or a progressive weakening and/or degeneration of muscles. The loss of muscle mass and/or the progressive weakening and/or degeneration of muscles can occur because of e.g., an unusually high rate of protein degradation, an unusually low rate of protein synthesis, or a combination of both. An unusually high rate of muscle protein degradation can occur due to muscle protein catabolism (i.e., the breakdown of muscle protein in order to use amino acids as substrates for gluconeogenesis). Muscle atrophy may also encompass significant loss in muscle strength. By "significant loss in muscle strength" is meant a reduction of strength in diseased, injured, or unused muscle tissue in a subject relative to the same muscle tissue in a control subject.

Muscle atrophy can have a genetic, organic or systemic origin. Major categories of inherited disorders primarily affecting the skeletal muscle tissue are muscular dystrophies and myopathies.

Muscle atrophy is a debilitating response, not only to activity, but also to many systemic diseases such as for example hyperuremia, chronic obstructive pulmonary disease, diabetes, sepsis, obesity, aids, cancer and heart failure. Generally, the loss of muscle mass is accompanied by a loss of muscle function and quality (i.e. the force generated by each volumetric unit of muscle tissue). "Skeletal muscle atrophy" as herein defined designates a reduction of muscle mass, caused by excessive protein degradation, and/or a progressive weakening and degeneration of skeletal muscles.

The expression "skeletal muscle atrophy" designates a loss of skeletal muscle mass and/or a progressive weakening and/or degeneration of skeletal muscles.

A muscle is a soft tissue found in most animals comprising muscle cells. Muscle cells contain protein filaments that can slide past one another and produce a contraction that changes both the length and shape of the muscle cell. Muscles function to produce force and motion. There are three types of muscles in the body: a) skeletal muscle (the muscle responsible for moving extremities and external areas of the bodies); b) cardiac muscle (the heart muscle); and c) smooth muscle (the muscle that is in the walls of arteries and bowel).

Skeletal muscle, or voluntary muscle, is generally anchored by tendons to bone and is generally used to effect skeletal movement such as locomotion or in maintaining posture. Although some control of skeletal muscle is generally maintained as an unconscious reflex (e.g., postural muscles or the diaphragm), skeletal muscles react to conscious control. Skeletal muscles are striated in that they contain sarcomeres that are packed into highly regular arrangements of bundles.

Skeletal muscle is further divided into two broad types: Type I (or "slow twitch") and Type II (or "fast twitch"). Type I muscle fibers are dense with capillaries and are rich in mitochondria and myoglobin, which gives Type I muscle tissue a characteristic red color. Type I muscle fibers can carry more oxygen and sustain aerobic activity using fats or carbohydrates for fuel. Type I muscle fibers contract for long periods of time but with little force. In large mammals, including humans, type II muscle fibers may be subdivided into two major subtypes (IIa and IIx) that vary in both contractile speed and force generated. Type II muscle fibers contract quickly and powerfully but fatigue very rapidly, and therefore produce only short, anaerobic bursts of activity before muscle contraction becomes painful.

The term "subject" or "patient" refers to an individual who requires detection and/or assessment of muscle atrophy, in particular skeletal muscle atrophy. The subject is at risk for or suffering from (skeletal) muscle atrophy. The subject may be either asymptomatic or suffering from symptoms not related to (skeletal) muscle atrophy. Typically, the subject may have one of the above mentioned systemic disease. The term "subject" or "patient" includes, but is not limited to mammals, either a human or a non-human mammal.

The term "biological sample" includes any biological sample from a subject. The biological sample may be a fluid sample. Typical examples of biological fluid samples usable in the context of the present invention may be selected from a blood, plasma, serum, urine, saliva and bone marrow aspirate sample. Preferably, the biological sample is a plasma, a peripheral blood or a serum sample. In the methods of the invention, sample may be used pure or diluted. A dilution of the sample may be useful to remove inhibitors that could interfere with the measure of the expression level of the biomarker of interest. A pre-enrichment of the sample may be performed to concentrate the biomarkers. The sample may be treated prior to use, and/or frozen or lyophilized, and/or used extemporaneously.

The expression "determining the expression level of" a biomarker in a sample, control or reference, as described herein shall refer to the detection and quantification of the presence of said biomarkers in the tested sample. For example, the concentration of the biomarkers in said samples may be directly quantified via measuring the amount of protein/polypeptide as present in the tested sample. However, also possible is to quantify the amount of biomarker indirectly via assessing the gene expression of the encoding gene of the biomarker, for example by quantification of the expressed mRNA encoding for the respective biomarker. The present invention shall not be restricted to any particular method for determining the level of a given biomarker, but shall encompass all means that allow for a quantification, or estimation, of the expression level of said biomarker, either directly or indirectly. "Level" in the context of the present invention is therefore a parameter describing the absolute amount of a biomarker in a given sample, for example as absolute weight, volume, or molar amounts; or alternatively "level" pertains to the relative amounts, for example and preferably the concentration of said biomarker in the tested sample, for example mol/l, g/l, g/mol etc. When analyzing gene expression using qRT-PCR, the "level of expression" of a target gene is expressed as a ΔΔCt value or as the number of mRNA molecules per µl. When analyzing gene expression using digital PCR, the "level of expression" of a target gene is expressed as copies of target per µl or as copies of target per droplet (CPD).

To quantify a difference in biomarker expression level, an expression ratio can be calculated. This differential expression refers to quantitative, as well as qualitative, difference in biomarker expression level between a subject with muscle atrophy and a reference value. The term "reduction" in the expression level of a biomarker refers to an expression value for at least one biomarker in the biological sample of a tested subject that is lower than the reference value for the same biomarker. Typically, a reduction in the expression level of a biomarker may correspond to a decrease of 5% or more, 10% or more, 20% or more, 30% or more, 40% or more, 50% or more, 60% or more, 70% or more, 80% or more, 90% or more of the expression level of the reference value for the same biomarker.

The term "reference value" can refer to a basal value corresponding to the median or mean of values (measured levels, quantities or concentrations) obtained with the biological sample(s) of a reference subject or population, typically of a healthy subject or of a population or cohort of healthy subjects, i.e. of subjects who do not suffer from muscle atrophy, in particular skeletal muscle atrophy. The reference value can also be a statistic or discriminating value/threshold, i.e. a value which has been determined by measuring the parameter in both a healthy control population and a population with (skeletal) muscle atrophy and /or suffering from any other disease, disorder or dysfunctional state which have a skeletal muscular atrophy as a secondary outcome. The threshold between "normal" and "atrophy" is defined as the limit between healthy subjects not suffering from muscle atrophy, in particular skeletal muscle atrophy, and diseased patients undergoing muscle atrophy, in particular skeletal muscle atrophy. The discriminating value identifies the diseased population with a specificity and/or a sensitivity, both predetermined by the skilled person, based on the analysis of the relation between the parameter's values and the known clinical data of the healthy control subject/population and of the diseased patient subject/population. The discriminating value determined in this manner is valid for the same experimental setup in future individual tests. The reference value can also be a value measured in a sample from a control subject. The "Reference value" can be substituted by a standard curve using known amounts (µmoles or copies) of the target biomarker(s).

The terms "diagnostic" and "diagnosing" refer to the detection or identification of the presence, stage, severity, progression, etc. of a pathology, disease, disorder or dysfunctional state, typically a (skeletal) muscle atrophy, or to the evaluation/assessment (dosing, comparison) of the severity or of the stage of such a muscle atrophy in a subject. Diagnostic may allow for early detection of the muscle atrophy, i.e. before the appearance of clinical signs, or intervenes to confirm the clinical observation made by a practitioner.

### Biomarkers

The invention discloses the identification of biomarkers that allow efficient diagnosis of muscle atrophy, in particular skeletal muscle atrophy. These biomarkers, have been selected from a set of about 1,500 mRNA identified by RNAseq approach in patients with lung cancer or kidney failure. A second round of selection based on criteria such as for example large amplitude of variation between healthy and sick subjects, good reproducibility from one subject to another, has led to the identification of a subset of 30 mRNAs. Among this subset, a dozen of mRNAs is sufficient, alone and/or in combination(s), to produce strong and specific diagnostic results of muscle atrophy, in particular skeletal muscle atrophy.

The sequence information of these biomarkers is publicly available and may be queried in the known NCBI Entrez gene or protein databases. The identifications and the nucleic acid and protein sequences for each of these biomarkers is provided below as well as in Table A.

**Table A: List of biomarkers**

| Gene Name | Entrez gene ID | Entrez protein ID | Gene description | SEQ ID # |
|---|---|---|---|---|
| ACTR6 | 64431 | Q9GZN1 | Actin-related protein 6 | SEQ ID NO: 1 |
| GIMAP2 | 26157 | Q9UG22 | GTPase IMAP family member 22 | SEQ ID NO: 2 |
| RCN2 | 5955 | Q14257 | Reticulocalbin - 2 | SEQ ID NO: 3 |
| RPL22L1 | 200916 | Q6P5R6 | 60S ribosomal protein L22-like 1 | SEQ ID NO: 4 |
| TRIAP 1 | 51499 | 043715 | TP53-regulated inhibitor of apoptosis 1 | SEQ ID NO: 5 |
| NIFK | 84365 | Q9BYG3 | MKI67 FHA domain-interacting nucleolar phosphoprotein | SEQ ID NO: 6 |
| APIP | 51074 | Q96GX9 | Methylthioribulose-1-phosphate dehydratase | SEQ ID NO: 7 |
| GEMIN6 | 79833 | Q8WXD5 | Gem-associated protein 6 | SEQ ID NO: 8 |
| RWDD1 | 51389 | Q9H446 | RWD domain-containing protein 1 | SEQ ID NO: 9 |
| ZNF613 | 79898 | Q6PF04 | Zinc finger protein 613 | SEQ ID NO: 10 |
| BPNT1 | 10380 | 095861 | 3'(2'),5'-bisphosphate nucleotidase 1 | SEQ ID NO: 11 |
| CCT2 | 10576 | P78371 | T-complex protein 1 subunit beta | SEQ ID NO: 12 |
| LYRM2 | 57226 | Q9NU23 | LYR motif-containing protein 2 | SEQ ID NO: 13 |

Within the context of the present invention, the expression "ACTR6 gene" designates preferably a human ACTR6 gene, particularly a nucleic acid molecule or sequence comprising (i) a sequence of gene #64431 or a sequence complementary thereto; (ii) a natural variant of a sequence of (i) such as a polymorphism; or (iii) a sequence having at least 90% identity, preferably at least 95, 96, 97, 98 or 99%, to a sequence of (i) or (ii). In a particular embodiment, the method comprises determining the presence, the absence or amount of at least one sequence encoded by an exon of the ACTR6 gene.

The same definition applies to any other gene quoted in the present application, by reference to the corresponding reference sequence in Table A.

Preferred biomarkers for use in the invention are ACTR6, GIMAP2 and RCN2. As disclosed in the examples, measuring the expression level of one of these biomarkers is sufficient to provide a reliable diagnosis of muscle atrophy, in particular skeletal muscle atrophy.

With regard to ACTR6, in a particular embodiment, the method preferably comprises measuring a target sequence consisting of or comprised in SEQ ID NO: 1 or its complementary strand. In another particular embodiment, the method preferably comprises measuring the protein identified under the accession number Q9GZN1.

With regard to GIMAP2, in a particular embodiment, the method preferably comprises measuring a target sequence consisting of or comprised in SEQ ID NO: 2 or its complementary strand. In another particular embodiment, the method preferably comprises measuring the protein identified under the accession number Q9UG22.

With regard to RCN2, in a particular embodiment, the method preferably comprises measuring a target sequence consisting of or comprised in SEQ ID NO: 3 or its complementary strand. In another particular embodiment, the method preferably comprises measuring the protein identified under the accession number Q14257.

The present invention shows that the above genes or their alternative isoforms are deregulated in subjects having muscle atrophy, in particular skeletal muscle atrophy, so that any sequence of an expression product encoded by these genes may be measured and correlated to muscle atrophy.

The combination of some of the previously described biomarkers appear to show a very high sensitivity and specificity in order to diagnose muscle atrophy, in particular skeletal muscle atrophy.

In preferred aspects, the invention comprises measuring the expression level of a combination of at least two biomarkers selected from ACTR6, GIMAP2 and RCN2.

In a more preferred aspect, the invention comprises measuring the expression level of the two biomarkers ACTR6 and GIMAP2. The results shown in the experimental section demonstrate that, by measuring the expression of ACTR6 and GIMAP2, the Area Under the ROC Curve is about 0.9736. Accuracy of the prediction of "skeletal muscle atrophy" is 95%.

In another preferred aspect, the invention comprises measuring the expression level of the two biomarkers ACTR6 and RCN2.

In another preferred aspect, the invention comprises measuring the expression level of the two biomarkers GIMAP2 and RCN2.

In another preferred aspect, the invention comprises measuring the expression level of the combination of the three biomarkers ACTR6, GIMAP2 and RCN2.

Furthermore, while the above three biomarkers provide highly reliable test, it is possible to further improve the method by combining said markers with further biomarkers identified by the inventors. Appropriate (secondary) diagnostic biomarkers suitable for use in the present invention, in addition to ACTR6, GIMAP2 and RCN2, are selected from RPL22L1, TRIAP1, NIFK, APIP, GEMIN6, RWDD1, ZNF613, BPNT1, CCT2 and LYRM2.

In one embodiment of the invention, the number of biomarkers comprises any one of the following numbers of biomarkers: 1 or more, 2 or more, 3 or more, 4 or more, 5 or more, 6 or more, 7 or more, 8 or more, 9 or more, 10 or more, 11 or more, 12 or more, or 13. In general the optimal panel size for the biomarkers of the invention is between 1 and 5, preferably between 3 and 5.

With regard to RPL22L1, in a particular embodiment, the method preferably comprises measuring a target sequence consisting of or comprised in SEQ ID NO: 4 or its complementary strand. In another particular embodiment, the method preferably comprises measuring the protein identified under the accession number Q6P5R6.

With regard to TRIAP1, in a particular embodiment, the method preferably comprises measuring a target sequence consisting of or comprised in SEQ ID NO: 5 or its complementary strand. In another particular embodiment, the method preferably comprises measuring the protein identified under the accession number 043715.

With regard to NIFK, in a particular embodiment, the method preferably comprises measuring a target sequence consisting of or comprised in SEQ ID NO: 6 or its complementary strand. In another particular embodiment, the method preferably comprises measuring the protein identified under the accession number Q9BYG3.

With regard to APIP, in a particular embodiment, the method preferably comprises measuring a target sequence consisting of or comprised in SEQ ID NO: 7 or its complementary strand. In another particular embodiment, the method preferably comprises measuring the protein identified under the accession number Q96GX9.

With regard to GEMIN6, in a particular embodiment, the method preferably comprises measuring a target sequence consisting of or comprised in SEQ ID NO: 8 or its complementary strand. In another particular embodiment, the method preferably comprises measuring the protein identified under the accession number Q8WXD5.

With regard to RWDD1, in a particular embodiment, the method preferably comprises measuring a target sequence consisting of or comprised in SEQ ID NO: 9 or its complementary strand. In another particular embodiment, the method preferably comprises measuring the protein identified under the accession number Q9H446.

With regard to ZNF613, in a particular embodiment, the method preferably comprises measuring a target sequence consisting of or comprised in SEQ ID NO: 10 or its complementary strand. In another particular embodiment, the method preferably comprises measuring the protein identified under the accession number Q6PF04.

With regard to BPNT1, in a particular embodiment, the method preferably comprises measuring a target sequence consisting of or comprised in SEQ ID NO: 11 or its complementary strand. In another particular embodiment, the method preferably comprises measuring the protein identified under the accession number 095861.

With regard to CCT2, in a particular embodiment, the method preferably comprises measuring a target sequence consisting of or comprised in SEQ ID NO: 12 or its complementary strand. In another particular embodiment, the method preferably comprises measuring the protein identified under the accession number P78371.

With regard to LYRM2, the method comprises measuring a target sequence consisting of or comprised in SEQ ID NO: 13 or its complementary strand. In another particular embodiment, the method preferably comprises measuring the protein identified under the accession number Q9NU23.

### Methods for determining biomarker expression level

According to a preferred aspect, determining the expression level of at least one biomarker selected from ACTR6, GIMAP2, RCN2, RPL22L1, TRIAP1, NIFK, APIP, GEMIN6, RWDD1, ZNF613, BPNT1, CCT2 and LYRM2 can be done at the nucleic acid and/or protein level.

When mRNA is chosen as the (or one of the) gene product whose levels are determined, this can be the total of all mRNA isoforms for the gene(s) under study, or one or more specific mRNAs. When protein is chosen as the (or one of the) gene product whose levels are determined, the same considerations apply: the total protein levels may be determined, or those of specific isoforms only (e.g. using an antibody against the different C-termini). Most particularly, all protein isoforms may be detected (e.g. using an antibody against a common epitope). Of note, it is contemplated as well that both mRNA and protein are determined. In this case, the isoforms to be detected can be all isoforms for both mRNA and protein, identical isoforms (wholly overlapping), or different isoforms (partly or not overlapping), depending on the setup of the experiment. With identical isoforms, it is meant that the mRNA isoform encodes for the corresponding protein isoform. However, for several genes it is known that the number of protein isoforms detected is generally lower than the number of possible mRNA transcripts (and thus of protein isoforms). When a target gene exists in several isoforms, one can choose to amplify the majority isoform, several or all isoforms of this gene.

According to a preferred embodiment, the method comprises determining the expression level of an RNA encoded by a target gene as defined above, preferably by selective amplification.

### Measure by selective amplification

Selective amplification of target genes is preferably performed using a primer, a pair of primers, or pairs of primers to amplify all or part of the target gene(s), i.e. the target nucleic acid(s), in the sample from a subject.

The term "primer" designates nucleic acid molecules that can form base pairs with a complementary template and serve to initiate amplification of the template in the presence of reagents (polymerase or reverse transcriptase) and four different nucleoside triphosphates (NTPs) at appropriate buffer solutions and temperatures.

The primer pairs of the present invention are carefully designed to maximize the sensitivity and specificity of the detection of the target genes. Primers are typically single-stranded nucleic acids, with a length comprised between 10 and 30 bases, preferably between 15 and 30 bases, more preferably between 15 and 25 bases. Primers can perfectly match with the targeted sequence in the target gene, or can have one or more mismatch with the targeted sequence when several isoforms of the target gene exist provided that the mismatch(s) does not impair the specific amplification of the targeted sequence. The primers of the present invention can incorporate additional features/modifications known in the art that do not alter the basic properties. Examples of such modifications include, but are not limited to, methylation, capping, substitution of one or more nucleotides, and uncharged linkages such as phosphonates, phosphotriesters, phosphoramidates or carbamates, phosphorothioates or phosphorodithioates. The nucleic acid sequence of the primer used in the present invention can also be modified using a label capable of directly or indirectly providing a detectable signal.

As used herein, the terms "amplification" and "amplify" encompass all methods for copying or reproducing a target nucleic acid sequence, thereby increasing the number of copies or amount of the nucleic acid sequence in a sample. The amplification may be exponential or linear, and the target nucleic acid may be DNA, cDNA or RNA. Sequences amplified in this manner are referred to herein as an "amplicon".

The method of the invention may comprise amplifying at least one target sequence from at least one biomarker selected from ACTR6, GIMAP2, RCN2, RPL22L1, TRIAP1, NIFK, APIP, GEMIN6, RWDD1, ZNF613, BPNT1, CCT2 and LYRM2 in a biological sample obtained from a subject. In a particular embodiment, the amplified target sequence (i.e. amplicon) is 70-250 nt, preferably 80-230 nt, more preferably 90-210 nt in length. Specific examples of primers suitable for conducting amplification(s) of the target biomarkers are provided in Table B below. These primers have been designed to bind target sequences of SEQ ID NOs: 1-13. Further primers can be designed to bind and amplify one or more of the target sequences of SEQ ID NOs: 1-13.

**Table B: List of primers for amplification of target sequence**

| Target gene | Primer sense | Primer sequence | SEQ ID # |
|---|---|---|---|
| ACTR6 | Forward | CGCCAAAATCGGTTACAGCC TCCCAATTCACCAAGTAGCCC | SEQ ID NO: 14 |
| | Reverse | | SEQ ID NO: 15 |
| GIMAP2 | Forward | GACCTGTGGGATCAGATGAAAG CTTCAGCCAAGGCTGTGTAA | SEQ ID NO: 16 |
| | Reverse | | SEQ ID NO: 17 |
| RCN2 | Forward | AACACTGCTCTGGATGATGC ACTCAAACCGGGACCTGAATC | SEQ ID NO: 18 |
| | Reverse | | SEQ ID NO: 19 |
| RPL22L1 | Forward | GGCAAAACTGGAAATCTCGGG ATGCAACCACTCGAAGCCA | SEQ ID NO: 20 |
| | Reverse | | SEQ ID NO: 21 |
| TRIAP 1 | Forward | GCTTCAATCGCTGGTTCGC | SEQ ID NO: 22 |
| | Reverse | TCTGAACACACTGCTGGTAGC | SEQ ID NO: 23 |
| NIFK | Forward | CGCAGGAAGATGTCGAGTTT GGGTTTCGTCAAGTAGGTTAGG | SEQ ID NO: 24 |
| | Reverse | | SEQ ID NO: 25 |
| APIP | Forward | CGCCATCGAAGAAGCTAAA GCAGCTTTAGAGTGGGTATG | SEQ ID NO: 26 |
| | Reverse | | SEQ ID NO: 27 |
| GEMIN6 | Forward | TGCATTTGTTCACGTCTGGAG AGTGATGGGGATGTGGTTCTTC | SEQ ID NO: 28 |
| | Reverse | | SEQ ID NO: 29 |
| RWDD1 | Forward | ATGCTGGAAACAACGTGGAG TTCATCATCCTCCAGCTCCAAG | SEQ ID NO: 30 |
| | Reverse | | SEQ ID NO: 31 |
| ZNF613 | Forward | CCTGCTCACACAAGTCAGGT ACAAGGCATGACAAGTGGGA | SEQ ID NO: 32 |
| | Reverse | | SEQ ID NO: 33 |
| BPNT1 | Forward | CACAACTACTCGATCCCATAGC CTTATTTCCTGCTCCTCCTACTC | SEQ ID NO: 34 |
| | Reverse | | SEQ ID NO: 35 |
| CCT2 | Forward | CTGGTGTCATGGCTATTGAG GTTCTGGGTGATCAAAGGTAG | SEQ ID NO: 36 |
| | Reverse | | SEQ ID NO: 37 |
| LYRM2 | Forward | ACCTGGAAGATTGGGCAAGA CCTTGAGCTGCATATTGCCTT | SEQ ID NO: 38 |
| | Reverse | | SEQ ID NO: 39 |

In a preferred aspect, the expression level of target biomarkers is determined at the nucleic acid level by detecting and/or quantifying target genes-encoding nucleic acid in the biological sample of a subject, such as for example RNA or cDNA.

In this particular aspect, determining the expression level of at least one biomarker comprises performing real time reverse transcriptase polymerase chain reaction (RT-PCR), quantitative real time reverse transcriptase polymerase chain reaction (qRT-PCR), digital PCR, RNAseq, microarrays, gene chips, nCounter Gene Expression Assay, Serial Analysis of Gene Expression (SAGE), Rapid Analysis of Gene Expression (RAGE), nuclease protection assays, Northern blotting, or any other equivalent gene expression detection techniques.

In some embodiments, sequences from two or more genes of interest are amplified in the same reaction vessel. In this case, the amplicon(s) could be detected by first size-separating the amplicons then detection of the size-separated amplicons. The separation of amplicons of different sizes can be accomplished by, for example, gel electrophoresis, column chromatography, denaturing HPLC, capillary electrophoresis, hybridization with a probe, or sequencing. These and other separation methods are well-known in the art. In one example, amplicons of about 10 to about 150 base pairs whose sizes differ by 10 or more base pairs can be separated, for example, on a 4% to 5% agarose gel, (a 2% to 3% agarose gel for about 150 to about 300 base pair amplicons) or a 6% to 10% polyacrylamide gel. The separated nucleic acids can then be stained with a dye such as ethidium bromide and the size of the resulting stained band or bands can be compared to a standard DNA ladder.

Alternatively, amplicons may be detected by hybridization with a specific probe. The term "probe" used herein refers to a nucleic acid molecule, complementary to a portion of the amplified target sequence (*i.e.* amplicon), consisting of several to several tens bases that can be specifically bound to a target sequence, and may be labeled to identify the existence of a specific amplified target sequence.

Useful labels include, e.g., fluorescent dyes (e.g., Cy5, Cy3, FITC, rhodamine, lanthamide phosphors, Texas red), 32P, 35S, 3H, 14C, 125I, 131I, electron-dense reagents (e.g., gold), enzymes, e.g., as commonly used in an ELISA (e.g., horseradish peroxidase, beta-galactosidase, luciferase, alkaline phosphatase), colorimetric labels (e.g., colloidal gold), magnetic labels (e.g., Dynabcads^{™}), biotin, dioxigenin, or haptens and proteins for which antisera or monoclonal antibodies are available. Other labels include ligands or oligonucleotides capable of forming a complex with the corresponding oligonucleotide. The label can be directly incorporated into the nucleic acid to be detected, or it can be attached to a probe (e.g., an oligonucleotide) or antibody that hybridizes or binds to the nucleic acid to be detected.

The real time quantitative RT-PCR measures PCR product accumulation through a dual-labeled fluorogenic probe. The probes employed in qRT-PCR are based on the principle of fluorescence quenching and involve a donor fluorophore (also named reported dye) at the 5' end, and a quenching moiety (also named quencher dye) at the 3' end. The term "quencher moiety" as used herein means a molecule that, in close proximity to a donor fluorophore, takes up emission energy generated by the donor and either dissipates the energy as heat or emits light of a longer wavelength than the emission wavelength of the donor. Fluorophores can be, but are not limited to, FAM, TAMRA, VIC, JOE, TET, HEX, ROX, RED610, RED670, NED, Cy3, Cy5, and Texas Red. Quenchers can be, but are not limited to, 6-TAMRA, BHQ-1,2,3 and MGB-NFQ. The choice of the fluorophore-quencher pair can be made so that the excitation spectrum of the quencher has an overlap with the emission spectrum of the fluorophore. An example is the pair FAM-TAMRA, FAM-MGB, VIC-MGB and so on. A skill artisan in the art will know how to recognize other suitable pairs.

The probes can be prepared in the form of single stranded DNA, double stranded DNA, RNA or hybrid DNA-RNA. The typical length of a probe is 10-60 nt, preferably 15-55 nt, more preferably 20-50 nt, more preferably 30-45 nt, even more preferably 10-30 nt.

Various probe formats can be used to perform real-time RT-PCR, such as for example TaqMan^{™}, Molecular Beacon^{™}, Scorpion^{™}, and LUX^{™} probes.

Another object of the present invention thus relates to a nucleic acid having anyone of the nucleotide sequences as described in Table B, composition comprising them as well as the use of these nucleic acids for diagnosing muscle atrophy, in particular skeletal muscle atrophy.

In particular, the present invention relates to oligonucleotide having a nucleic acid sequence selected from the group consisting of SEQ ID NOs: 14-39.

The oligonucleotides may be used as primer, in particular primer pair, for amplifying one or more specific amplicon of the above identified biomarkers. Typically, a primer pair consists in a forward primer and a reverse primer that both hybridize to the target sequence. In the context of the present invention, an oligonucleotide primer pair comprises a forward oligonucleotide primer and a reverse oligonucleotide primer capable of binding and amplifying at least one target sequences of SEQ ID Nos: 1-13, wherein the oligonucleotides have the nucleic acid sequence of SEQ ID NOs: 14-39.

In particular aspects, the invention relates to sets of oligonucleotide primer pairs specific for at least one biomarker selected from ACTR6, GIMAP2, RCN2, RPL22L1, TRIAP1, NIFK, APIP, GEMIN6, RWDD1, ZNF613, BPNT1, CCT2 and LYRM2. As used herein, the term "specific" means that the primers bind only to one of the biomarkers of the invention, with negligible binding to other biomarkers of the invention or to other analytes in the biological sample being analyzed. This ensures that the integrity of the diagnostic assay and its result using the biomarkers of the invention is not compromised by additional binding events. Example of oligonucleotide primer pairs suitable for amplifying each of the target biomarkers are listed in Table B. As an example, the set of oligonucleotide primer comprising the forward primer of SEQ ID NO:14 and the reverse primer of SEQ ID NO: 15 allow amplification of ACTR6.

In some embodiments of the methods of the invention herein described, probes can be used to detect at least one the above mentioned biomarkers or to quantify the obtained amplicons.

Amplified nucleic acids for each of the target sequences may be detected simultaneously (i.e., in the same reaction vessel) or individually (i.e., in separate reaction vessels). In some embodiments, the amplified DNA is detected simultaneously in a multiplex format, using distinguishably-labeled, gene-specific oligonucleotide probes, each which hybridizes to different target sequences. In case the assay is performed in parallel in separate reaction vessels, the gene-specific oligonucleotide probes could have the same label.

To correct for (normalize away) both differences in the amount of RNA assayed and variability in the quality of the RNA used the assay can optionally incorporate analysis of the expression of certain reference genes (or "normalizing genes"), including well known housekeeping genes, such as GAPDH (Entrez gene ID#P04406, SEQ ID NO: 40), 36B4/RPLP0 (Entrez gene ID#P05388, SEQ ID NO: 41), ACTB (Entrez gene ID#P60709, SEQ ID NO: 42), etc. Specific examples of primers suitable for conducting amplification(s) of the reference genes are provided in Table C below. These primers have been designed to bind target sequences of SEQ ID NOs: 40-42. Further primers can be designed to bind and amplify one or more of the target sequences of SEQ ID NOs: 40-42.

**Table C: List of primers for amplification of reference gene**

| Target gene | Primer sense | Primer sequence | SEQ ID # |
|---|---|---|---|
| GAPDH | Forward | AGGTCATCCCTGAGCTAGACG TGGGTGTCGCTGTTGAAGTC | SEQ ID NO: 43 |
| | Reverse | | SEQ ID NO: 44 |
| 36B4/RPLP0 | Forward | TGGTCATCCAGCAGGTGTTCGA ACAGACACTGGCAACATTGCGG | SEQ ID NO: 45 |
| | Reverse | | SEQ ID NO: 46 |
| ACTB | Forward | TCCCTGGAGAAGAGCTACGA AGCACTGTGTTGGCGTACAG | SEQ ID NO: 47 |
| | Reverse | | SEQ ID NO: 48 |

Alternatively, an absolute quantitation of target biomarker may be obtained thanks to calibrated standards (calibration curve) or by digital PCR. Amounts of RNA or DNA may be determined by comparing the results to a standard curve produced by real-time PCR of serial dilutions of a known amount of RNA or DNA. The absolute or relative copy number of a target molecule in the Polymerase Chain Reaction (PCR) amplification can be determined by comparing the cycle threshold (Ct) value with a standard curve, with the cycle threshold (Ct) value of a reference nucleic acid or with an absolutely quantitated standard nucleic acid. For absolute quantitation, the range of expression of the same at least one biomarker is expressed as the number of molecules present in the biological sample.

### Measuring polypeptide

According to another preferred aspect, the method comprises determining the expression level of a polypeptide encoded by a target gene as defined above.

In this particular aspect, the expression level of target biomarker is determined at the protein level in the biological sample of a subject. In other words, the expression level of target biomarker is determined by detecting ACTR6, GIMAP2, RCN2, RPL22L1, TRIAP1, NIFK, APIP, GEMIN6, RWDD1, ZNF613, BPNT1, CCT2 and LYRM2 polypeptides in the biological sample of a subject.

Measuring or assaying a polypeptide in a sample can be performed by any known technique, most notably by means of a specific ligand than binds the polypeptide encoded by a target gene, for example an antibody, an antibody fragment or a derivative thereof. The term "antibody" as used herein includes, but is not limited to: polyclonal, monoclonal, bispecific, humanized or chimeric antibodies, single chain antibodies, Fab fragments and F(ab')2 fragments, fragments produced by a Fab expression library, anti-idiotypic (anti-Id) antibodies and epitope-binding fragments of any of the above. The term "epitope" refers to a site on an antigen to which an antibody binds. An epitope can be formed from contiguous amino acids or noncontiguous amino acids juxtaposed by tertiary folding of one or more proteins. The term "antibody" as used herein also refers to immunoglobulin molecules and immunologically-active portions of immunoglobulin molecules, i.e., molecules that contain an antigen binding site that specifically binds an antigen. The immunoglobulin molecules of the disclosure can be of any class (e. g., IgG, IgE, IgM, IgD and IgA) or subclass of immunoglobulin molecule.

Non-limiting examples of antibody fragments include (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment, which consists of a VH domain; and (vi) an isolated complementarity determining region (CDR). Furthermore, although the two domains of the Fv fragment, VL and VH, are coded for by separate genes, they can be joined, using recombinant methods, by a synthetic or natural linker that enables them to be made as a single protein chain in which the VL and VH regions pair to form monovalent molecules (known as single chain Fv (scFv)). Any VH and VL sequences of specific single chain antibodies can be linked to human immunoglobulin constant region cDNA or genomic sequences, in order to generate expression vectors encoding complete IgG molecules or other isotypes. VH and VL can also be used in the generation of Fab, Fv or other fragments of immunoglobulins using either protein chemistry or recombinant DNA technology. Other forms of single chain antibodies, such as diabodies are also encompassed.

"F(ab')2" and "Fab'" moieties can be produced by treating immunoglobulin (monoclonal antibody) with a protease such as pepsin and papain, and includes an antibody fragment generated by digesting immunoglobulin near the disulfide bonds existing between the hinge regions in each of the two H chains. For example, papain cleaves IgG upstream of the disulfide bonds existing between the hinge regions in each of the two H chains to generate two homologous antibody fragments in which an L chain composed of VL (L chain variable region) and CL (L chain constant region), and an H chain fragment composed of VH (H chain variable region) and CHγ1 (γ1 region in the constant region of H chain) are connected at their C terminal regions through a disulfide bond. Each of these two homologous antibody fragments is called Fab'. Pepsin also cleaves IgG downstream of the disulfide bonds existing between the hinge regions in each of the two H chains to generate an antibody fragment slightly larger than the fragment in which the two above-mentioned Fab' are connected at the hinge region. This antibody fragment is called F(ab')2.

The Fab fragment also contains the constant domain of the light chain and the first constant domain (CH1) of the heavy chain Fab' fragments differ from Fab fragments by the addition of a few residues at the carboxyl terminus of the heavy chain CH1 domain including one or more cysteine(s) from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known.

"Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the VH-VL dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

"Single-chain Fv" or "sFv" antibody fragments comprise a VH, a VL, or both a VH and VL domain of an antibody, wherein both domains are present in a single polypeptide chain. In some aspects, the Fv polypeptide further comprises a polypeptide linker between the VH and VL domains which enables the sFv to form the desired structure for antigen binding.

Preferably, the ligand is a specific antibody, antibody fragment or derivative thereof that binds to an epitope of ACTR6, GIMAP2, RCN2, RPL22L1, TRIAP1, NIFK, APIP, GEMIN6, RWDD1, ZNF613, BPNT1, CCT2 or LYRM2 polypeptides.

Commercial antibodies are available for some of the target polypeptides, such as for example mouse monoclonal antibodies sc-514988 (anti-ACTR6 antibody), ABIN525619 (anti-GIMAP2 antibody), SAB1404305 (anti-RCN2 antibody), ABIN2586231 (anti-NIFK antibody), sc-376666 (anti-APIP antibody), sc-514919 (anti-GEMIN6 antibody), sc-514496 (anti-RWDDl antibody), sc-393185 (anti-BPNTl antibody), sc-374152 (anti-CCT2 antibody); and rabbit polyclonal antibodies HPA038587 (anti-ACTR6 antibody), HPA013589 (anti-GIMAP2 antibody), AB104516 (anti-RCN2 antibody), HPA056207 (anti-RPL22L1 antibody), HPA043640 (anti-TRIAPl antibody), HPA021188 (anti-APIP antibody), HPA035727 (anti-GEMIN6 antibody), HPA028712 (anti-RWDD1 antibody), HPA026833 (anti-ZNF613 antibody), HPA048461 (anti-BPNT1 antibody), HPA003198 (anti-CCT2 antibody) and HPA063932 (anti-LYRM2 antibody).

Other specific antibodies of target polypeptides may be produced by conventional techniques, notably by immunization of a non-human mammal with an immunogen comprising part or all of the target polypeptide, and recovery of the antibodies (polyclonal) or cells production monoclonal antibodies. The immunogen can be produced by synthesis, or by expression, in a suitable host, of a nucleic acid target such as defined previously. In common monoclonal antibody methods, the synthesized antibody genes can be expressed and purified by inserting them into a virus-based or non-viral expression vector for antibody expression. Both prokaryotic, such as for example E.Coli,, Bacillus subtilis, Salmonella, Serratia, or Pseudomonas; and eukaryotic cells, such as for example HEK293, CHO or CHO-DG44, are suitable to produce an antibody. To express the antibody of the present invention, other microorganisms, for example, yeasts can be used, and an insect cell combined with a baculovirus vector may be also used. Such antibodies, monoclonal or polyclonal, as well as fragments or derivatives thereof with the same antigenic specificity, constitute further objects of the present invention, as well as the use thereof to diagnose muscle atrophy, in particular skeletal muscle atrophy.

In this particular aspect of the invention, determining the expression level of at least one biomarker comprises performing immunoassay, immunosorbent assay (ELISA), radioimmunoassay (RIA), mass spectrometry, western blotting, flow cytometry, or any other equivalent protein detection techniques.

### Methods

Expression level of the herein described biomarkers has been highly correlated to the atrophying muscle program that occurs in diseased subjects. Provided herein are efficient methods for assessing the muscular atrophy status in a subject. The invention can be used, for example, for one or more of:
(i) diagnosing, prognosing and/or evaluating, a subject having muscle atrophy, in particular skeletal muscle atrophy; (ii) assessing the efficacy of a treatment to reverse (skeletal) muscle atrophy; (iii) assessing the occurrence or the risk of occurrence of a disease in a patient diagnosed with (skeletal) muscle atrophy; and/or (iv) helping in selecting, adapting or changing treatment in a diseased subject.

In another aspect, the present invention relates to an *in vitro* or *ex vivo* method for diagnosing muscle atrophy, in particular skeletal muscle atrophy, in a subject, comprising determining the expression level of at least one biomarker selected from ACTR6, GIMAP2, RCN2, RPL22L1, TRIAP1, NIFK, APIP, GEMIN6, RWDD1, ZNF613, BPNT1, CCT2 and LYRM2 in a biological sample obtained from a subject; wherein a reduction in expression level of said at least one biomarker as compared to a reference value is indicative of muscle atrophy, in particular skeletal muscle atrophy, in the subject.

In a particular aspect, the subject is a mammal, preferably a human subject, even more preferably an adult human subject, such as for example older than (but not limited to) 18 years.

The subject may be tested whatever his/her sex. The subject may be apparently healthy or suffering from any disease or disorder, such as for example hyperuremia, sepsis, stroke, spinal cord injury, cancer, diabetes, obesity, neuromuscular disease, chronic inflammatory disease, chronic kidney disease, heart failure (such as congestive heart failure or congestive cardiac failure), lung disease (such as chronic obstructive pulmonary disease), and acute critical illness chronic renal failure, or any other disease. In a particular aspect, the subject suffers from a cancer, in particular lung cancer, small intestine cancer or stomach cancer. In another particular aspect, the subject suffers from a chronic disease, in particular chronic renal failure.

In a particular aspect, the biological sample is a fluid sample, preferably selected from peripheral blood, plasma and serum.

The expression level of the biomarkers identified by the inventors may be decreased in a subject suffering from muscle atrophy, in particular skeletal muscle atrophy, as compared to a healthy subject.

In a particular embodiment, the *in vitro* or *ex vivo* method for diagnosing muscle atrophy, in particular skeletal muscle atrophy, in a subject, comprises determining the expression level of at least one biomarker selected from ACTR6, GIMAP2, RCN2, or a combination thereof, in a biological sample obtained from the subject; wherein a reduction in expression level of said at least one biomarker as compared to a refence value is indicative of muscle atrophy in the subject.

The combination of ACTR6, GIMAP2 and RCN2 biomarkers as intended by the present invention may be a combination of two biomarkers : ACTR6 and GIMAP2, ACTR6 and RCN2, GIMAP2 and RCN2, or of the three biomarkers ACTR6, GIMAP2 and RCN2.

While ACTR6, GIMAP2 and RCN2 biomarkers provide highly reliable test, it is possible to further improve the method by combining said biomarkers with further biomarkers identified by the inventors. Appropriate (secondary) diagnostic biomarkers suitable for use in the present invention, in addition to ACTR6, GIMAP2 and RCN2, are selected from RPL22L1, TRIAP1, NIFK, APIP, GEMIN6, RWDD1, ZNF613, BPNT1, CCT2 and LYRM2.

In another particular embodiment, the *in vitro* or *ex vivo* method for diagnosing muscle atrophy, in particular skeletal muscle atrophy, in a subject, further comprises_determining the expression level of at least one further biomarker selected from the group consisting of RPL22L1 (SEQ ID NO:4), TRIAP1 (SEQ ID NO:5), NIFK (SEQ ID NO:6), APIP (SEQ ID NO:7), GEMIN6 (SEQ ID NO:8), RWDD1 (SEQ ID NO:9), ZNF613 (SEQ ID NO: 10), BPNT1 (SEQ ID NO:11), CCT2 (SEQ ID NO:12) and LYRM2 (SEQ ID NO:13).

Accuracy of a method aiming to diagnose an event, or not, is best described by its receiver-operating characteristics (ROC). Each point on the ROC plot represents a sensitivity/specificity pair corresponding to a particular decision threshold. Dependent on a desired confidence interval, a threshold can be derived from the ROC curve allowing for the diagnosis or prediction for a given event with a proper balance of sensitivity and specificity, respectively. Accordingly, the reference to be used for the method of the present invention, i.e. a threshold which allows to discriminate between subjects who have muscle atrophy and those who don't have muscle atrophy can be generated, preferably, by establishing a ROC analysis for said cohort as described above and deriving one or more threshold amounts or concentrations therefrom. Dependent on a desired sensitivity and specificity for a diagnostic method, the ROC plot allows deriving adjustable suitable thresholds. It will be understood that an optimal sensitivity is desired for excluding muscle atrophy (i.e., a rule out) whereas an optimal specificity is envisaged for a subject to be identified as having muscle atrophy (i.e. a rule in).

As for the three preferred biomarkers, an absolute quantitation of the herein described at least one further biomarker in the biological sample of the subject is obtained thanks to calibrated standards (calibration curve) or by digital PCR. For absolute quantitation, the range of expression of the same at least one biomarker is expressed in number of molecules present in the biological sample. When a calibration curve is used, the expression level of the herein described at least one further biomarker in the biological sample of the subject is compared to a statistic or discriminating value/threshold that enables diagnosis of muscle atrophy, in particular skeletal muscle atrophy.

Once the diagnosis of muscular atrophy has been made, the subject can be treated to recover all or part of the loss of muscle mass. A variety of approaches, referred as "countermeasures" or "treatment", can be implemented in attempts to reverse (skeletal) muscle atrophy and functional loss (Chopard et al., J. Cell. Mol. Med., (2009) 13 (9b): 3032-3050). These approaches can be grouped into three distinct classes: exercise and physical training, nutritional aids and use of drugs. Dynamic and maximal resistance exercises are associated with a decrease in the induction of MAFbx and MURF1 (*i.e.* 2 muscle-specific atrophy-related genes), thus allowing for maintenance of muscle mass notably by counteracting the decrease in contractile, cytoskeletal and membrane-associated proteins. Amino acid supplementation can also promote protein synthesis. Among essential amino acids, leucine appears to mediate most of the effects of protein/amino acid intake on protein metabolism and is recognized for its potential anabolic effects. Growth factors, such as for example IGFs, FGFs and TGF-β, are also known to exert a profound influence over muscle genes expression by promoting proliferation of satellite cells in muscle. Compounds with antioxidant properties, such as for example soy protein, vitamin C, vitamin E, N-acetylcysyteine and curcumin exert their effect by modulating the expression of muscle proteolysis-related genes. More recently, ursolic acid and tomatidine were found to reduce skeletal muscle atrophy in mouse models by stimulating skeletal muscle hypertrophy (Ebert et al., Physiology (2019), 34: 232-239).

The diagnosis of muscle atrophy, in particular skeletal muscle atrophy, may allow a follow-up of the recovery of the muscle mass and/or strength.

In another aspect, the invention relates to an *in vitro* or *ex vivo* method for assessing the efficacy of a treatment to reverse muscle atrophy in a subject, comprising:
(i) determining the expression level of at least one biomarker selected from ACTR6, GIMAP2, RCN2, RPL22L1, TRIAP1, NIFK, APIP, GEMIN6, RWDD1, ZNF613, BPNT1, CCT2 and LYRM2 in a first biological sample from the subject obtained prior to said treatment or at an early stage of said treatment; and
(ii) determining the expression level of the same at least one biomarker in a second biological sample obtained from the subject obtained during, at a later stage, or after said treatment; wherein an expression level of the at least one biomarker as measured in step (ii) above the expression level of the same at least one biomarker as measured in step (i) is indicative that the treatment is effective.

The expression "assessing the efficacy of a treatment to reverse muscle atrophy" means for the purpose of the present invention to observe muscle atrophy, in particular skeletal muscle atrophy, progression or regression in a subject who receives countermeasures (i.e. treatment) to reverse muscle atrophy. In other words, the subject during the therapy is regularly monitored for the effect of the applied treatment, which allows the medical practitioner to estimate at an early stage during the therapy whether the prescribed treatment is effective or not to reverse muscle atrophy, and therefore to adjust the treatment accordingly. Muscle atrophy, in particular skeletal muscle atrophy, is reversed if at least one symptom of the muscle atrophy is reduced, alleviated, terminated, slowed or prevented. Muscle mass may be determined by the following approaches: DXA (Dual-energy X-Ray densitometer), Skeletal Muscle Index, Skeletal muscle area, Masseter Cross Sectional Area (CSA), Psoas CSA, Total Psoas area, L4 vertebral index, CT scan, etc. (for more details, see Xiao-Ming Zhang, BMC Geriatrics, 2021).

In another preferred aspect, the invention relates to an *in vitro* or *ex vivo* method for assessing the efficacy of a treatment to reverse muscle atrophy in a subject, comprising:
(i) determining the expression level of at least one biomarker selected from ACTR6, GIMAP2, RCN2, or a combination thereof, in a first biological sample from the subject obtained prior to said treatment or at an early stage of said treatment; and
(ii) determining the expression level of the same at least one biomarker, or the combination thereof, in a second biological sample obtained from the subject obtained during, at a later stage, or after said treatment; wherein an expression level of the at least one biomarker, or the combination thereof, as measured in step (ii) above the expression level of the same at least one biomarker as measured in step (i) is indicative that the treatment is effective.

All the possible combinations of the three biomarkers are herein described.

In still another preferred aspect, the method further comprises in step (i) determining the expression level of at least one further biomarker selected from RPL22L1, TRIAP1, NIFK, APIP, GEMIN6, RWDD1, ZNF613, BPNT1, CCT2 and LYRM2.

Many diseases or disorder are associated with skeletal muscle atrophy, muscle weakness, and general muscle fatigue. Skeletal muscle wasting (i.e., atrophy) is associated with increased morbidity and mortality and a decreased quality of life. In addition to muscle wasting and associated weakness, muscle fatigue is one of the most common and debilitating symptoms experienced by subjects suffering from cancer. Chronic inflammatory diseases appear to have direct, deleterious effects on skeletal muscles of the trunk and limbs, leading to a decline in performance and hence fatigue. In neurological disorders, however, fatigue is often present before changes in muscle performance are observed. The diagnosis of muscle atrophy, in particular skeletal muscle atrophy, may also be useful in determining if the subject encountering muscle atrophy has, or is suspected of having or at risk of having a disease or a disorder.

In another aspect, the present invention relates to an *in vitro* or *ex vivo* method for assessing the occurrence or the risk of occurrence of a disease in a subject, comprising assessing the presence of a muscle atrophy in the subject by a method herein described; wherein the presence of muscle atrophy is indicative of the occurrence or the risk of occurrence of a disease in the subject.

In a particular aspect of this method, the presence of muscle atrophy, in particular skeletal muscle atrophy, is established by determining the expression level of at least one biomarker selected from ACTR6, GIMAP2, RCN2, RPL22L1, TRIAP1, NIFK, APIP, GEMIN6, RWDD1, ZNF613, BPNT1, CCT2 and LYRM2.

In another particular aspect of this method, the presence of muscle atrophy, in particular skeletal muscle atrophy, is established by determining the expression level of at least one biomarker selected from ACTR6, GIMAP2, RCN2 or a combination thereof. In this particular aspect, determining the expression level of at least one further biomarker selected from RPL22L1, TRIAP1, NIFK, APIP, GEMIN6, RWDD1, ZNF613, BPNT1, CCT2 and LYRM2 may be used to improve the diagnosis of a muscle atrophy, in particular skeletal muscle atrophy.

Fatigue resulting from or preceding muscle atrophy may limit treatment options in diseases or disorders. In fact, perceived fatigue has been rated to have a more negative impact on daily activities and quality of life than other disease or disorder related symptoms. Fatigue occurs as a consequence of both the disease or disorder itself and as a side effect of disease or disorder treatments. Hence, it may be necessary to modulate treatment with therapeutic agents or therapeutic regimen in subjects suffering from a disease or a disorder.

In another aspect, the present invention relates to an *in vitro* or *ex vivo* method for helping in selecting, adapting or changing treatment in a subject suffering from a disease, comprising:
(i) assessing the presence of a muscle atrophy, in particular skeletal muscle atrophy in the subject by a method herein described; and
(ii) taking into account the presence of muscle atrophy, in particular skeletal muscle atrophy, for selecting, adapting or changing treatment in the subject suffering from a disease.

In a particular aspect of this method, the presence of muscle atrophy, in particular skeletal muscle atrophy, is established by determining the expression level of at least one biomarker selected from ACTR6, GIMAP2, RCN2, RPL22L1, TRIAP1, NIFK, APIP, GEMIN6, RWDD1, ZNF613, BPNT1, CCT2 and LYRM2.

In another particular aspect of this method, the presence of muscle atrophy, in particular skeletal muscle atrophy, is established by determining the expression level of at least one biomarker selected from ACTR6, GIMAP2 and RCN2 or a combination thereof. In this particular aspect, determining the expression level of at least one further biomarker selected from RPL22L1, TRIAP1, NIFK, APIP, GEMIN6, RWDD1, ZNF613, BPNT1, CCT2 and LYRM2 may be used to improve the diagnosis of a muscle atrophy, in particular skeletal muscle atrophy.

In other further particular aspects, the *in vitro* or *ex vivo* method for helping in selecting, adapting or changing treatment in a subject suffering from a disease further comprises a step (iii) of selecting a therapy for muscle atrophy, wherein said therapy comprises drugs (pharmacological agents such as glucocorticoids, anti-inflammatory drugs), nutritional agents (such as dietary supplements, ursolic acid, tomatidine), exercise, or a combination thereof.

In yet another aspect, the present invention also relates to a method for diagnosing and treating muscle atrophy, in particular skeletal muscle atrophy, in a subject, comprising:
(i) determining the level of expression of at least one biomarker selected from ACTR6, GIMAP2, RCN2, RPL22L1, TRIAP1, NIFK, APIP, GEMIN6, RWDD1, ZNF613, BPNT1, CCT2 and LYRM2 in a biological sample obtained from the subject; wherein a reduction in expression level of the at least one biomarker as compared to a reference value is indicative of muscle atrophy, in particular skeletal muscle atrophy, in the subject; and
(ii) treating the subject identified in step (i) as having muscle atrophy, in particular skeletal muscle atrophy, with drugs (pharmacological agents such as glucocorticoids, anti-inflammatory drugs), nutritional agents (such as dietary supplements, ursolic acid, tomatidine), exercise, or a combination thereof.

By "method for treating" is meant a process that is intended to produce a beneficial change in the condition of an individual, e.g., mammal, especially human. A beneficial change can include one or more of: restoration of muscle mass and/or strength, limitation or retardation of a muscle atrophy, or prevention, limitation or retardation of deterioration of a patient's condition, disease or disorder. In particular, as used herein, the term "treatment" (also "treat" or "treating") refers to any countermeasures that partially or completely alleviates, ameliorates, relieves, inhibits, delays onset of, reduces severity of and/or reduces incidence of muscle atrophy caused by a particular disease, disorder, and/or condition. Such treatment may be of a subject who does not exhibit signs of the muscle atrophy, and/or of a subject who exhibits only early signs of muscle atrophy. Alternatively or additionally, such treatment may be of a subject who exhibits one or more established signs of the muscle atrophy.

The step (i) of the method can also be performed using a standard curve used as a calibrator. When a calibration curve is used, the expression level of the herein described at least one biomarker in the biological sample of the subject is compared to a statistic or discriminating value/threshold that enables diagnosis of muscle atrophy, in particular skeletal muscle atrophy.

In a particular aspect, the presence of muscle atrophy, in particular skeletal muscle atrophy, in the subject is established by determining in step (i) the expression level of at least one biomarker selected from ACTR6, GIMAP2, RCN2, or a combination thereof. In this particular aspect, determining the expression level of at least one further biomarker selected from RPL22L1, TRIAP1, NIFK, APIP, GEMIN6, RWDD1, ZNF613, BPNT1, CCT2 and LYRM2 may be used to improve the diagnosis of a muscle atrophy, in particular skeletal muscle atrophy, in the subject.

### Kits

In another aspect, the present invention relates to a kit suitable comprising means suitable for measuring the expression level of at least one biomarker selected from ACTR6, GIMAP2, RCN2, RPL22L1, TRIAP1, NIFK, APIP, GEMIN6, RWDD1, ZNF613, BPNT1, CCT2 and LYRM2 in a biological sample of a subject. Kit generally comprises reagents, such as for example nucleic acid extraction/purification reagents, amplification reagents; containers; and optionally a leaflet of instructions for use. The reagents may be in the same or separate containers. The user may use any portion of the reagents to perform the reaction, following the instructions.

In a particular aspect, the kit for measuring the expression level of at least one biomarker selected from ACTR6, GIMAP2, RCN2, RPL22L1, TRIAP1, NIFK, APIP, GEMIN6, RWDD1, ZNF613, BPNT1, CCT2 and LYRM2, in a biological sample from a subject comprises at least two oligonucleotides having a nucleic acid sequence selected from the group consisting of SEQ ID NOs: 14-39, and optionally reagents for performing amplification reaction and/or instructions for use. Specific example of pairs of oligonucleotides chosen for amplifying at least one biomarker selected from ACTR6, GIMAP2, RCN2, RPL22L1, TRIAP1, NIFK, APIP, GEMIN6, RWDD1, ZNF613, BPNT1, CCT2 and LYRM2 correspond to the forward and reverse primers listed in Table B.

In another particular aspect, the kit comprises oligonucleotide primer pairs suitable for measuring the expression of at least one biomarker selected from ACTR6, GIMAP2 and RCN2, or a combination thereof. In this particular aspect, the oligonucleotides have the nucleic acid sequence of SEQ ID NOs: 14-19, wherein oligonucleotides of SEQ ID NO: 14 and 15 enable amplification of ACTR6, oligonucleotides of SEQ ID NO: 16 and 17 enable amplification of GIMAP2 and oligonucleotides of SEQ ID NO: 18 and 19 enable amplification of RCN2 biomarker.

In another particular aspect, the kit may further comprises oligonucleotides suitable for measuring the expression level of at least one further biomarkers selected from RPL22L1, TRIAP1, NIFK, APIP, GEMIN6, RWDD1, ZNF613, BPNT1, CCT2 and LYRM2. In this particular embodiment, the kit comprises oligonucleotides having a nucleotide sequence of SEQ ID NOs: 20-39, wherein oligonucleotides of SEQ ID NO: 20 and 21 enable amplification of RPL22L1, oligonucleotides of SEQ ID NO: 22 and 23 enable amplification of TRIAP1, oligonucleotides of SEQ ID NO: 24 and 25 enable amplification of NIFK, oligonucleotides of SEQ ID NO: 26 and 27 enable amplification of APIP, oligonucleotides of SEQ ID NO: 28 and 29 enable amplification of GEMIN6, oligonucleotides of SEQ ID NO: 30 and 31 enable amplification of RWDD1, oligonucleotides of SEQ ID NO: 32 and 33 enable amplification of ZNF613, oligonucleotides of SEQ ID NO: 34 and 35 enable amplification of BPNT1, oligonucleotides of SEQ ID NO: 36 and 37 enable amplification of CCT2 and oligonucleotides of SEQ ID NO: 38 and 39 enable amplification of LYRM2 biomarker respectively.

The invention also relates to the use of kits as described above on a biological sample obtained from a subject, for *in vitro* or *ex vivo* diagnosing muscle atrophy, preferably skeletal muscle atrophy; for assessing the efficacy of a treatment to reverse muscle atrophy, preferably skeletal muscle atrophy; for assessing the occurrence or the risk of occurrence of a disease in a subject diagnosed as having (skeletal) muscle atrophy and/or for helping in selecting, adapting or changing treatment in a subject suffering from a disease.

The following examples are provided in order to demonstrate and further illustrate certain preferred aspects of the present invention and are not to be construed as limiting the scope thereof.

### EXAMPLES

### MATERIALS AND METHODS

### 1 - Biological samples

Whole blood samples were collected from patients of PROMETHE and PHD cohorts on a total of 83 subjects. 7 patients have renal failure, 14 patients have lung cancer, 49 subjects are hemodialyzed patients and 13 subjects are used as control or healthy subjects. Table 1 below provides main parameters of the subjects enrolled in this study.

**Table 1: Characteristics of the patients**

| | **Control/ PROMETHE** | | **Renal failure /PROMETHE** | | **Lung cancer /PROMETHE** | | **Hemodialyzed/ PHD** | |
|---|---|---|---|---|---|---|---|---|
| **Age (years)** | Mean (SD) 71+1 0 | Range 52-89 | Mean (SD) 70 + 9 | Range 58-83 | Mean (SD) 65+10 | Range 42-79 | Mean (SD) 68+12 | Range 34-89 |
| **Sex (M/F)** | M7/F6 | | M6/F1 | | M9/F5 | | M 25 / F 13 | |

2.5 ml of blood sample were collected in a PAXgene^{®} Blood RNA Tube (PreAnalytiX, Cat. No. / ID: 762165, distributed by Qiagen) and stored upright and at room temperature (18 to 25°C) for a minimum of 2 hours and a maximum of 72 hours before processing or placing in the refrigerator (2 to 8°C) or in the freezer (-20°C or -70/-80°C).

### 2 - Blood RNA extraction, purification and concentration

Thaw PAXgene^{®} Blood RNA Tube containing blood samples at room temperature (18 to 25° C), for approximately 2 hours. Then thaw PAXgene^{®} Blood RNA Tube containing blood samples at room temperature (18 to 25° C), for about 2 hours, the time required for complete lysis of blood cells. Total RNA was extracted using PAXgene Blood miRNA Kit (PreAnalytiX, Cat. No. / ID: 763134, distributed by Qiagen) according to the manufacturer's procedure, except that buffer BM5 is substituted by RNase free water in the final RNA elution step. Briefly, PAXgene Blood RNA Tubes are first centrifuged to pellet the samples, which are then washed with water and resuspended in Buffer BM1. After digestion in Buffer BM2 with proteinase K, the samples are homogenized by centrifugation through PAXgene Shredder spin columns. Isopropanol is added to the samples to optimize binding conditions, and the samples are then centrifuged through PAXgene RNA spin columns, where total RNA >18 nucleotides (including miRNA) binds to the PAXgene silica-membrane. The bound RNA is subjected to DNase digestion to remove genomic DNA contamination and washed with Buffer BM3 followed by Buffer BM4. Pure RNA is then eluted in RNAse-free water. The extracted RNAs (80 µl) are denatured in a water bath at 65°C for 5 minutes, placed immediately in ice, and then stored at -80 °C.

The extracted RNAs are then further purified and concentrated using the RNeasy^{®} MinElute^{®} Cleanup kit (Cat. No. / ID: 74204, Qiagen) according to the manufacturer's procedure.

### 3 - RNAseq

Gene Expression Analysis (mRNA analysis) was performed on Human blood samples. Illumina Ribo-Zero rRNA removal kit was first used for rRNA depletion. Globin depletion was also performed as recommended for RNA samples. PolyA mRNA were purified using oligo-dT linked to magnetic beads, fragmented and random primed. cDNA synthesis was followed by end-repair, phosphorylation (5' ends) and A-tailing (3' ends). Proprietary adapters were ligated and the cDNA fragments were amplified by PCR and sequenced (2x150 bp) using an Illumina platform (13-21 Million reads per sample).

### 4 - qRT-PCR

The purified and concentrated RNAs obtained with the RNeasy^{®} MinElute^{®} Cleanup kit is denatured in a water bath at 65°C for 5 minutes and then put on ice to prevent renaturation. Genomic DNA removal and reverse transcription of RNAs into cDNAs are done using QuantiTect^{®} Reverse Transcription Kit (Cat. No. / ID: 205311 or 205313, Qiagen) according to the manufacturer's procedure. Briefly, the purified RNA samples are incubated in gDNA Wipeout Buffer at 42°C for 2 minutes to effectively remove contaminating genomic DNA. After genomic DNA elimination, the RNA samples are ready for reverse transcription using a master mix prepared from Quantiscript Reverse Transcriptase, Quantiscript RT Buffer, and RT Primer Mix. The entire reaction takes place at 42°C for 30 minutes and is then inactivated at 95°C for 3 minutes. cDNA samples are stored at - 20°C until qPCR.

Semi-quantitative qPCR is performed on cDNA samples using the SsoADV Univer SYBR^{®} Green Supermix kit (Cat. No. / ID: 1725274, Bio-Rad) according to the manufacturer's procedure. Firstly, cDNA samples are diluted to 1/50. For each sample (1 well), the volume of the reaction mixture is 10 µl. It includes 0.7 µl of 10 µM sense primer, 0.7 µl of 10 µM anti-sense primer, 7 µl of Mix iQ and 1.6 µl of RNase free water. On a 96-well PCR plate, 4 µl of cDNA samples diluted to 1/50 are distributed in each well with 10 µl of the reaction mixture. Each cDNA sample is distributed in duplicate. The semi-quantitative qPCR reactions are carried out on a CFX96 Bio-Rad Laboratories thermal cycler. The calculations are carried out using the comparative method ΔΔCt using houskeeping genes.

### 5 - Bioinformatic analysis of mRNA expression data

Sequence reads were trimmed to remove possible adapter sequences and nucleotides with poor quality using Trimmomatic v.0.36. The trimmed reads were mapped to the Homo sapiens GRCh38 reference genome available on ENSEMBL using the STAR aligner v.2.5.2b. The STAR aligner is a splice aligner that detects splice junctions and incorporates them to help align the entire read sequences. BAM files were generated as a result of this step. Below are the statistics of mapping the reads to the reference genome. Unique gene hit counts were calculated by using feature Counts from the Subread package v.1.5.2. Only unique reads that fell within exon regions were counted. Since a strand-specific library preparation was performed, the reads were strand-specifically counted.

### 6 - Statistical analysis

Statistical analysis were performed using R software (https://www.R-project.org). The tests were two-sided, with a Type I error set at α = 0.05. P-value were adjusted using Benjamini-Hoschberg method. Differential expression analysis was performed using linear model using the Limma Voom R package. Gene whose expression was above 0.5 CPM in at least all the samples of the same condition were removed. Normalization was performed using the TMM method. The RNAs significantly increased or decreased in a concordant manner in lung cancer and hemodialysis patients compared to the healthy controls were selected. A PLS model was then constructed using the mixomic R package to predict the expression matrix from the proteomic analysis of muscle biopsies with the total blood RNA expression matrix. The first 30 RNAs selected to build the model were kept. The RNA expression data measured by qRT-PCR were then used to model the membership of the patient group (sick or healthy) using PLS-DA. The ability of the models to predict group membership was assessed with the area under the receiver operating characteristic curves.

### RESULTS

Several mRNAs present in the whole blood of patients have a level of expression directly related to the presence of muscle atrophy, regardless of the pathology. Around 1,500 mRNAs have been identified by RNAseq in patients with lung cancer or kidney failure. The RNAseq approach identifies all the mRNAs in one sample but is too expensive to be routinely applicable in hospital. Therefore, the 30 best candidates were selected (large amplitude of variation between healthy and sick patients, good reproducibility from one patient to another, etc.) and verified by RT-qPCR test. Inventors thus selected among the 30 preselected mRNAs identified, those which were the most discriminating between healthy and sick patients.

Whole blood RNAs whose expression levels was best correlated with the common proteomic changes observed on muscle biopsies in humans in the two different catabolic states (hemodialysis n = 7 and lung cancer n = 7 vs control n = 7) were first identified. Differential analysis of blood transcriptome by RNAseq was coupled with skeletal muscle proteomic analysis by mass spectrometry to identify a series of candidate genes (PLS Sparse Regression Algorithm of the MixOmic R package).

qRT-PCR was performed to verify RNAseq result on the PROMETHE cohort (renal failure n = 7 and lung cancer n = 14 vs control n = 13) and a cohort of hemodialyzed patients n = 38 (PHD cohort). Logistic regression was used to evaluate the ability of potential biomarkers to identify a catabolic condition on a derivation cohort (renal failure n = 7 patients, lung cancer n = 7 patients and control n = 7 patients from the PROMETHE cohort) and validated on a different validation cohort (hemodialysis n = 38 patients from the PHD cohort, lung cancer = 7 patients, control n = 7 patients from PROMETHE cohort). Results obtained are summarized in Table 2

**Table 2: ROC parameters**

| RNA | AUC ROC derivation cohort | p | AUC ROC validation cohort | p |
|---|---|---|---|---|
| ACTR6 | 0.9444 | 0.003647587 | 1.00 | 0 |
| GIMAP2 | 0.8333 | 0.01363636 | 0.9628 | 0.0008406088 |
| RCN2 | 0.8472 | 0.009729237 | 0.9442 | 0.003306807 |
| APIP | 0.9028 | 0.006235971 | 0.9488 | 0.001569961 |
| BPNT1 | 0.9444 | 0.001182 | 0.9442 | 0.00124237 |
| CCT2 | 0.7639 | 0.01566358 | 0.9302 | 0.001529784 |
| ZNF613 | 0.8788 | 0.007208448 | 0.907 | 0.004259188 |
| TRIAP 1 | 0.7222 | 0.02570146 | 0.8857 | 0.006251037 |
| RWDD1 | 0.8472 | 0.01600028 | 0.8698 | 0.005643205 |
| LYRM2 | 0.9167 | 0.004671717 | 0.8512 | 0.007771511 |
| GEMIN6 | 0.8472 | 0.01318042 | 0.8326 | 0.009282753 |
| NIFK | 0.8194 | 0.01961981 | 0.6837 | 0.02370805 |
| RPL22L1 | 0.8889 | 0.00617284 | 0.614 | 0.02277679 |

Using the qRT-PCR data, a partial least square discriminant analysis (PLS-DA) was performed using all the patients (PROMETHE + PHD) for the prediction of having a muscle atrophy due to a pathology (either cancer or renal deficiency). First patients were randomly divided in a derivation cohort (n = 21) and a validation cohort (n = 52).

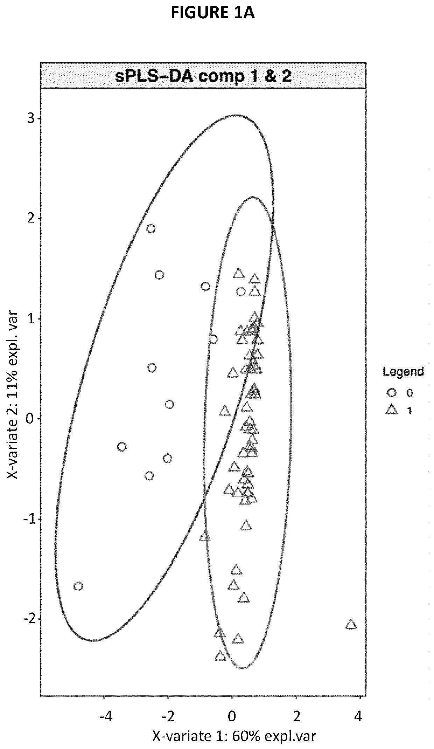

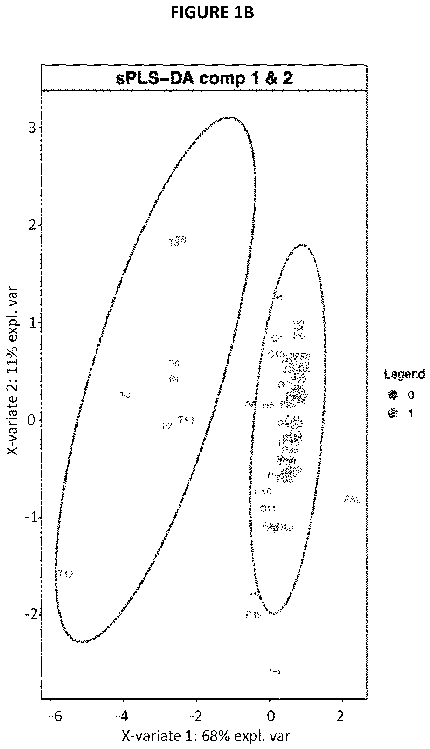

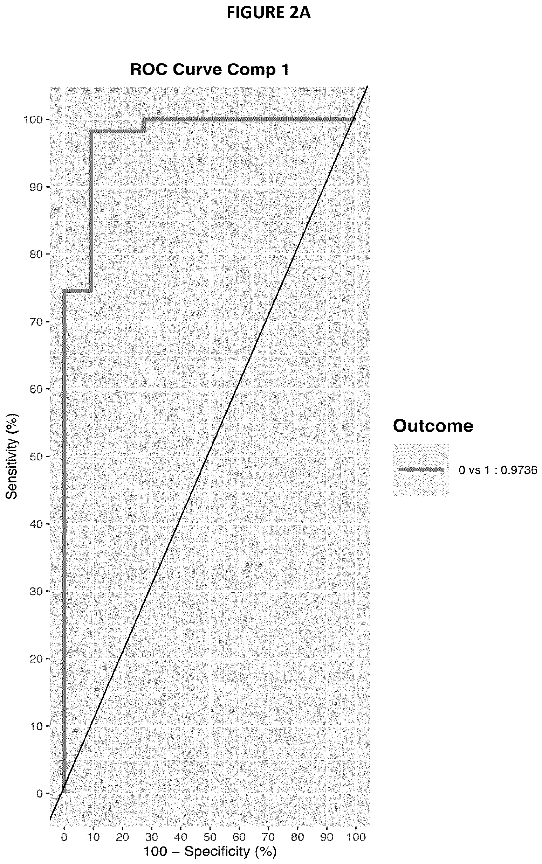

To select the most predictive or discriminative features that help classify the samples, inventors performed sparse Partial Least Square-Discriminant Analysis (sPLS-DA) using the qRT-PCR data of both the PROMETHE and PHD patients, with (Figure 1A) or without (Figure 1B) 4 outliers. Inventors found that a 1-component analysis including ACTR6 and GIMAP2 was enough to predict that the patient belongs to the muscle atrophy/pathology group. AUC of ROC is 0.9736 (Figure 2A). Accuracy of the prediction of "skeletal muscle atrophy" in the validation cohort is 95%. In addition, RCN2 behaved like ACTR6.

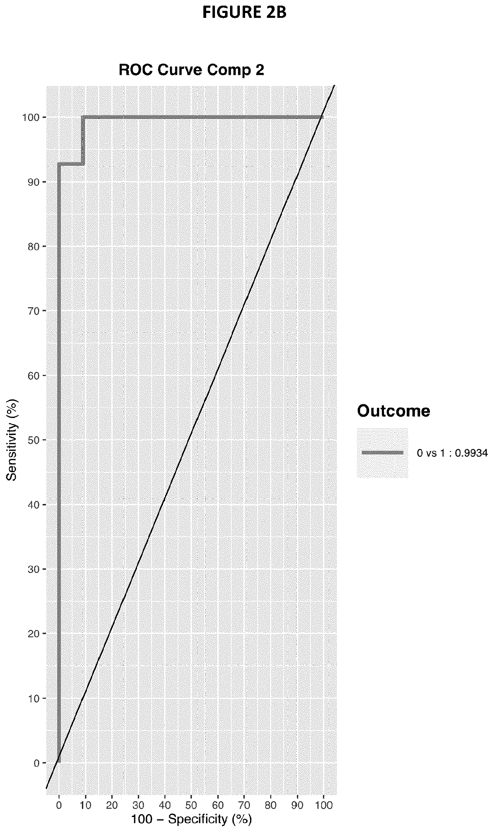

In a 2-components model NIFK, RPL22L1, CCT2, GEMIN6 and RCN2 were selected. AUC of ROC is 0.9934 (Figure 2B). Accuracy of the prediction of "skeletal muscle atrophy" in validation cohort is 95%.

## Claims

1. Use of at least one gene or its gene product selected from ACTR6 (SEQ ID NO:1), GIMAP2 (SEQ ID NO:2), RCN2 (SEQ ID NO:3), RPL22L1 (SEQ ID NO:4), TRIAP1 (SEQ ID NO:5), NIFK (SEQ ID NO:6), APIP (SEQ ID NO:7), GEMIN6 (SEQ ID NO:8), RWDD1 (SEQ ID NO:9), ZNF613 (SEQ ID NO:10), BPNT1 (SEQ ID NO:11), CCT2 (SEQ ID NO:12) and LYRM2 (SEQ ID NO:13) as a biomarker for *in vitro* or *ex vivo* detection of muscle atrophy.

2. An *in vitro* or *ex vivo* method for diagnosing muscle atrophy in a subject, comprising determining the expression level of at least one biomarker selected from ACTR6 (SEQ ID NO:1), GIMAP2 (SEQ ID NO:2), RCN2 (SEQ ID NO:3), RPL22L1 (SEQ ID NO:4), TRIAP1 (SEQ ID NO:5), NIFK (SEQ ID NO:6), APIP (SEQ ID NO:7), GEMIN6 (SEQ ID NO:8), RWDD1 (SEQ ID NO:9), ZNF613 (SEQ ID NO:10), BPNT1 (SEQ ID NO:11), CCT2 (SEQ ID NO:12) and LYRM2 (SEQ ID NO:13) in a biological sample obtained from the subject; wherein a reduction in expression level of said at least one biomarker as compared to a reference value is indicative of muscle atrophy in the subject.

3. The method of claim 2, wherein the at least one biomarker is selected from ACTR6, GIMAP2 and RCN2, or a combination thereof, and wherein a reduction in expression level of said at least one biomarker, or a combination thereof, as compared to a reference value is indicative of muscle atrophy.

4. The method of claim 3, which comprises determining the expression level of one of the following combinations of biomarkers : ACTR6 and GIMAP2, ACTR6 and RCN2, GIMAP2 and RCN2, or ACTR6 and GIMAP2 and RCN2.

5. The method of claim 3 or 4, which method further comprises determining the expression level of at least one further biomarker selected from RPL22L1, TRIAP1, NIFK, APIP, GEMIN6, RWDD1, ZNF613, BPNT1, CCT2 and LYRM2.

6. An *in vitro* or *ex vivo* method for assessing the efficacy of a treatment to reverse muscle atrophy in a subject, comprising the steps of:
(i) determining the expression level of at least one biomarker selected from ACTR6, GIMAP2, RCN2, RPL22L1, TRIAP1, NIFK, APIP, GEMIN6, RWDD1, ZNF613, BPNT1, CCT2 and LYRM2 in a first biological sample from the subject obtained prior to said treatment or at an early stage of said treatment; and
(ii) determining the expression level of the same at least one biomarker in a second biological sample from the subject obtained during, at a later stage, or after said treatment; wherein an expression level of the at least one biomarker as measured in step (ii) above the expression level of the same at least one biomarker as measured in step (i) is indicative that the treatment is effective.

7. An *in vitro* or *ex vivo* method for helping in selecting, adapting or changing treatment in a subject suffering from a disease, comprising the steps of:
(i) assessing the presence of muscle atrophy in the subject by a method according to any one of claims 2 to 5; and
(ii) taking into account the presence of muscle atrophy for selecting, adapting or changing treatment in the subject suffering from a disease.

8. The *in vitro* or *ex vivo* method of claim 7, further comprising a step (iii) of selecting a therapy for muscle atrophy, wherein said therapy comprises drugs (pharmacological agents such as glucocorticoids, anti-inflammatory drugs), nutritional agents (such as dietary supplements, ursolic acid, tomatidine), exercise, or a combination thereof.

9. The *in vitro* or *ex vivo* method of any one of claims 2 to 8, wherein the expression level of the at least one biomarker is determined at the nucleic acid level.

10. The *in vitro* or *ex vivo* method of claim 9, wherein the expression level of at least one biomarker is determined using real time reverse transcriptase polymerase chain reaction (RT-PCR), quantitative real time reverse transcriptase polymerase chain reaction (qRT-PCR), digital PCR, RNAseq, microarrays, gene chips, nCounter Gene Expression Assay, Serial Analysis of Gene Expression (SAGE), Rapid Analysis of Gene Expression (RAGE), nuclease protection assays, Northern blotting, or any other equivalent gene expression detection technique.

11. The method of any one of claims 2 to 10, wherein the subject is a mammal, preferably a human subject, even more preferably an adult human subject.

12. The method of any one of claims 2 to 11, wherein the biological sample is a fluid sample, preferably selected from peripheral blood, plasma and serum.

13. Oligonucleotide having a nucleic acid sequence selected from the group consisting of SEQ ID NOs: 14-39.

14. A kit comprising at least two oligonucleotides of claim 13 for amplifying of at least one biomarker selected from ACTR6, GIMAP2, RCN, RPL22L1, TRIAP1, NIFK, APIP, GEMIN6, RWDD1, ZNF613, BPNT1, CCT2 and LYRM2, and optionally reagents for performing amplification reaction and/or instructions for use.

15. Use of a kit of claim 14 for diagnosing muscle atrophy; for assessing the efficacy of a treatment to reverse muscle atrophy ; for assessing the occurrence or the risk of occurrence of a disease in a subject and/or for helping in selecting, adapting or changing treatment in a subject suffering from a disease.
